Europäisches Patentamt

European Patent Office

Office européen des brevets

(11) Publication number: **0 216 117**
**A2**

# EUROPEAN PATENT APPLICATION

(21) Application number: 86111305.8

(22) Date of filing: 15.08.86

(51) Int. Cl.⁴: **C 12 N 15/00**

(30) Priority: 20.08.85 JP 180984/85
03.07.86 JP 155041/86

(43) Date of publication of application:
01.04.87 Bulletin 87/14

(84) Designated Contracting States:
AT BE CH DE FR GB IT LI LU NL SE

(71) Applicant: Takeda Chemical Industries, Ltd.
27, Doshomachi 2-chome Higashi-ku
Osaka-shi Osaka, 541(JP)

(72) Inventor: Oshima, Yasuji
6-5, Hiyoshidai 2-bancho
Takatsuki Osaka 569(JP)

(72) Inventor: Araki, Hiroyuki
12-302, 35 Yamadahigashi 1-chome
Suita Osaka 564(JP)

(72) Inventor: Kaneko, Yoshinobu
8-10, Tarumicho 2-chome
Suita Osaka 564(JP)

(74) Representative: von Kreisler, Alek, Dipl.-Chem. et al,
Deichmannhaus am Hauptbahnhof
D-5000 Köln 1(DE)

(54) Novel DNA and use thereof.

(57) The present invention relates to a DNA having a promoter activity of PHO81 gene regulating the production of phosphatase, and which is obtainable from *Saccharomyces cerevisiae.*; a DNA bearing a structural gene downstream from the above PHO81 promoter; a transformant containing a DNA bearing the above PHO81 promoter; a transformant containing a DNA bearing the above PHO81 promoter and a structural gene downstream from the PHO81 promoter; and a process for obtaining a gene product which includes culturing a transformant containing a DNA bearing the above PHO81 promoter and a structural gene located downstream therefrom in a suitable medium until the gene product is formed and recovering the gene product from the culture.

Pharmacologically important proteinous materials may be efficiently produced with the use of the above-described novel and potent promoter obtained from yeast which is a eukaryotic microorganism.

**0216117**

## NOVEL DNA AND USE THEREOF

BACKGROUND OF THE INVENTION

<u>Field of the Invention</u>

This invention relates to a novel DNA containing a promoter obtained from yeast and to an application thereof in genetic engineering.

<u>Prior Art</u>:

With widespread utilization of DNA recombination techniques, it becomes now possible to produce useful polypeptides using prokaryotes or eukaryotes. <u>Escherichia coli</u> has thus far been employed on large scale production of polypeptides. However, the use of eukaryotes is desired for the production of polypeptides particularly important in pharmacology. Yeasts, a group of eukaryotic microorganisms, have a number of similarities with mammalian cells and, therefore, are advantageous for use in the expression of genes coding for mammalian proteins. Further, yeasts do not contain endotoxin in their cells. Yeasts are easily cultivated. Culture of yeasts has been made from the past on a large, industrial scale, and its safety is confirmed. Additionally, a number of studies have been made to clarify their genetic biological mechanism. All the above circumstances surrounding yeasts have led the utilization thereof as host organisms in genetic engineering.

Several yeast vectors for gene cloning are known at present. There are, however, known few yeast promoters capable of effective expressing foreign genes.

Two kinds of acid phosphatases and two kinds of alkaline phosphatases are known to exist in a lysate of a strain of yeast Saccharomyces cerevisiae. The acid phosphatases are found on the surface of cells. The production of one of the acid phosphatases is suppressed by an inorganic phosphate, while the other acid phosphatase is constitutively produced. One of the alkaline phosphatases is a repressible one whose production is repressed by an inorganic phosphate and which has a wide substrate specificity. The other alkaline phosphatase is a specific p-nitrophenylphosphatase whose substrate is only p-nitrophenylphosphate, and which is constitutively produced. The mutants, pho5, pho4, pho2 and pho81 which lack repressible acid phosphatase activity were isolated. The PHO5 gene is a structural gene for the repressible acid phosphatase, whereas the PHO4, PHO2 and PHO81 genes are genes which produce proteins regulating the expression of the repressible acid phosphatase structural gene PHO5. Further, the PHO4 and PHO81 genes serve to regulate the expression of repressive alkaline phosphatase structural gene similar to PHO5.

Various yeast vectors for gene cloning are known and may be utilized at present. In order to express effectively native or endogeneous genes and foreign or exogeneous genes, it is necessary to select a potent yeast promoter suitable for

- 3 -

**0216117**

the host organisms to be used.

<u>Brief Description of the Drawings:</u>

Fig. 1 is a restriction map of a plasmid pAC4 having an insertion of a DNA fragment bearing the PHO81 gene;

Fig. 2 is a schematic representation of a restriction maps for a DNA fragment and derivatives thereof bearing the PHO81 gene and the PHO81 promoter cloned according to the present invention, and their ability complement the PHO81 mutation;

Fig. 3 is a representation showing the identification of the PHO81 transcription products;

Fig. 4 represents a strategy for the base sequence of a cloned DNA fragment bearing PHO81;

Fig. 5 shows the nucleotide sequence of the promoter region of the PHO81 gene;

Fig. 6 is a scheme for constructing pAC430 plasmid ;

Fig. 7 is a scheme for constructing a recombinant DNA for the exprpession of adr type hepatitis B virus surface antigen gene; and

Fig. 8 is a scheme for constructing a lacZ expression plasmid; and

Fig. 9 shows the DNA sequence containing the PHO81 gene.

<u>Summary of the Invention:</u>

The present inventors have given their attention to a promoter of the PHO81 gene (hereinafter referred to as the PHO81 promoter), the PHO81 gene is a regulatory gene for expression of the structural genes of the repressible acid and alkaline phosphatases of yeast. Thus, the present inventors have cloned the PHO81 gene, determined the restriction enzyme cleavage map for the cloned DNA and determined the nucleotide sequence of the promoter region thereof. As a result, it has been found that the promoter is a novel DNA and is suitably used for efficiently expressing both foreign and native genes. The present invention has been made on the basis of the foregoing studies.

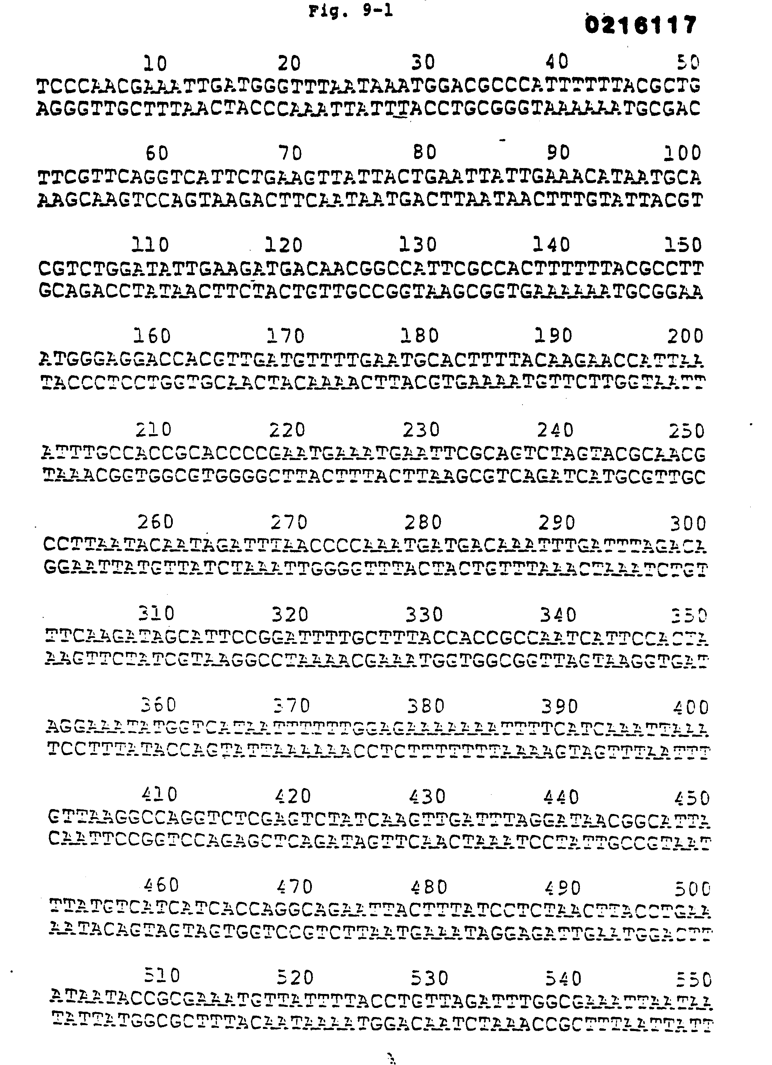

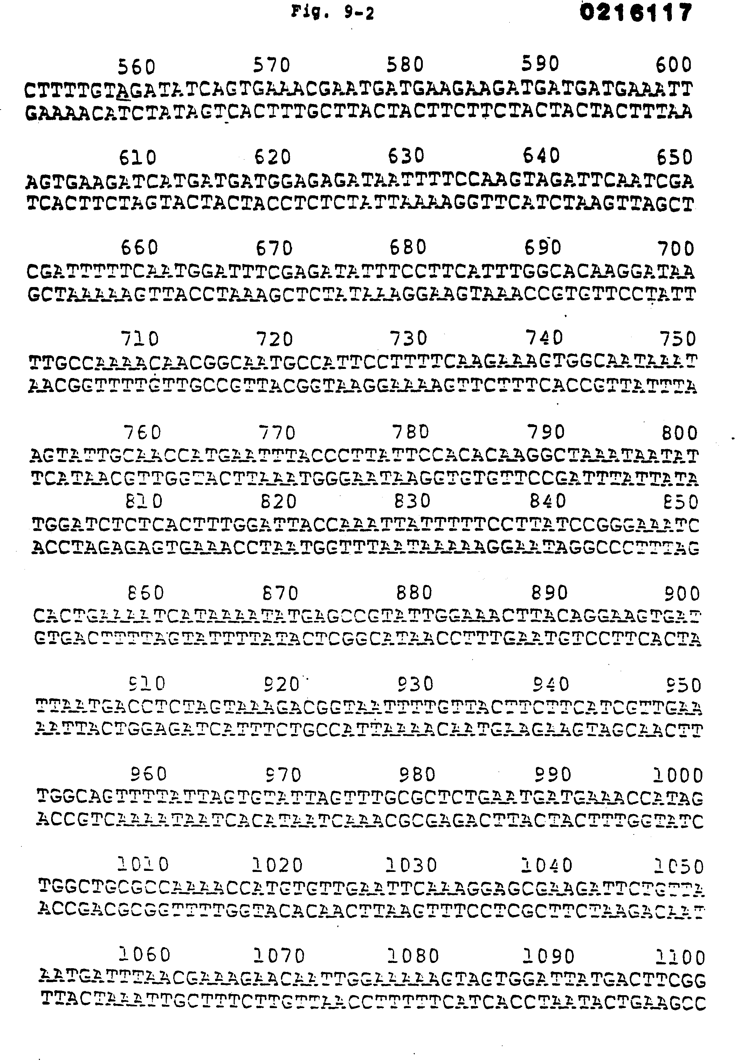

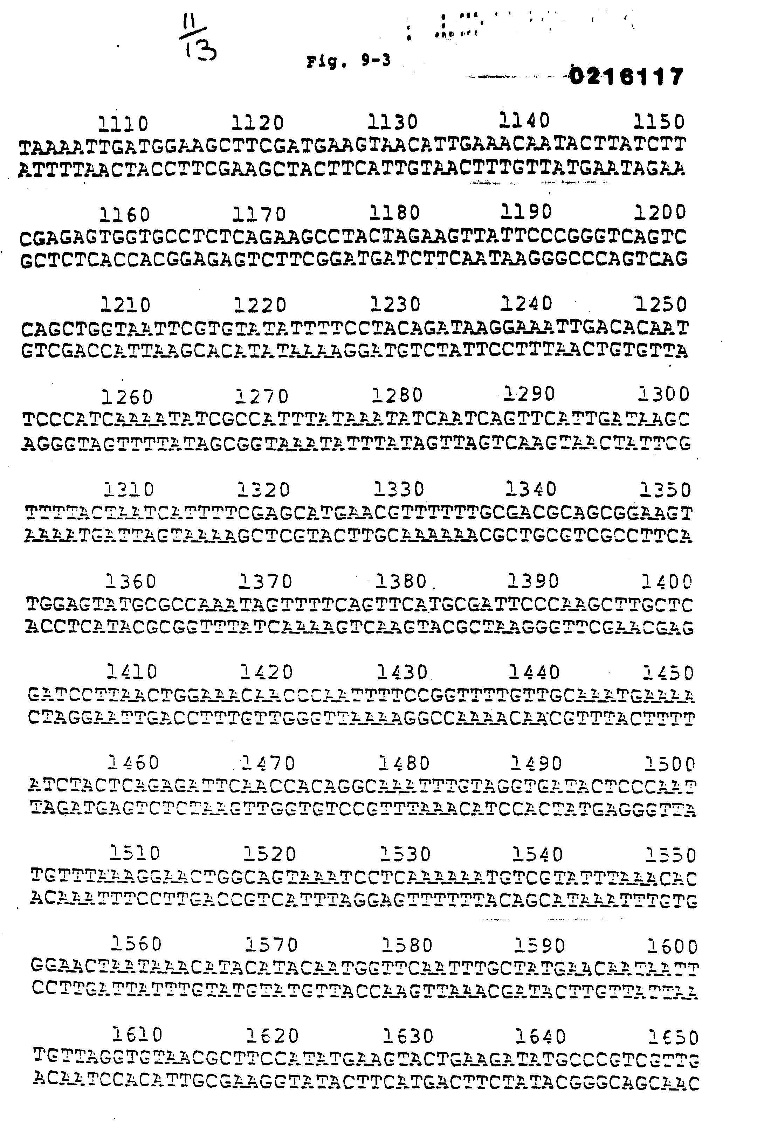

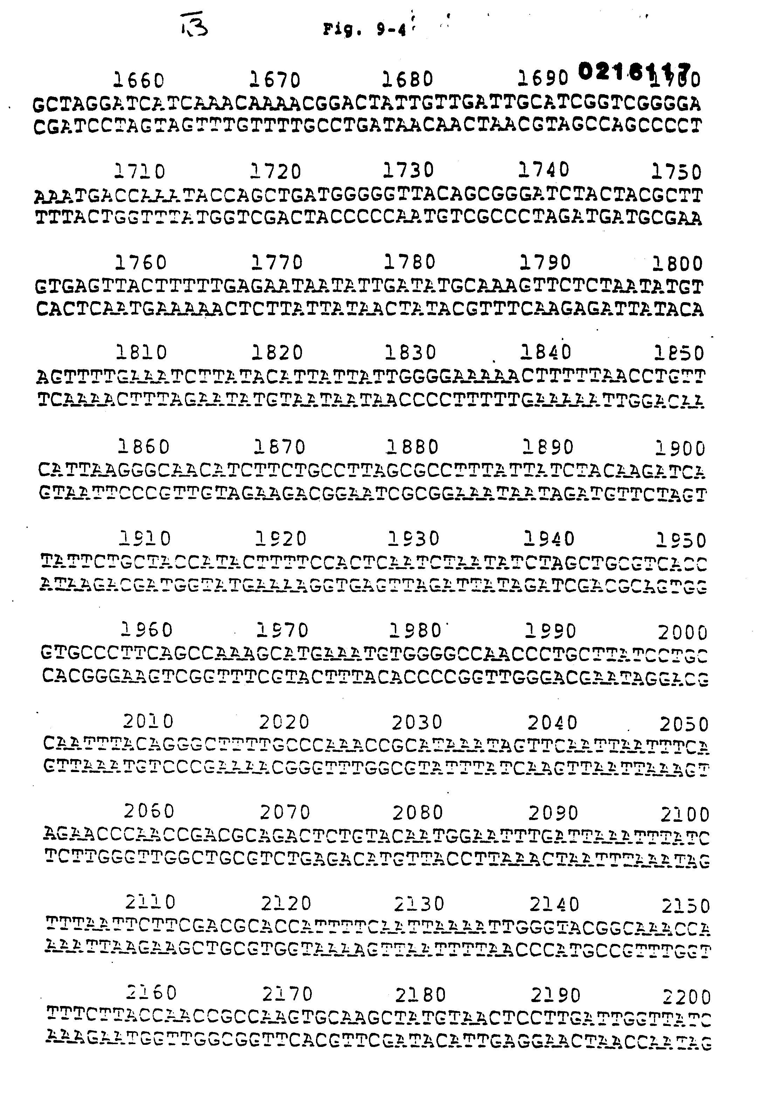

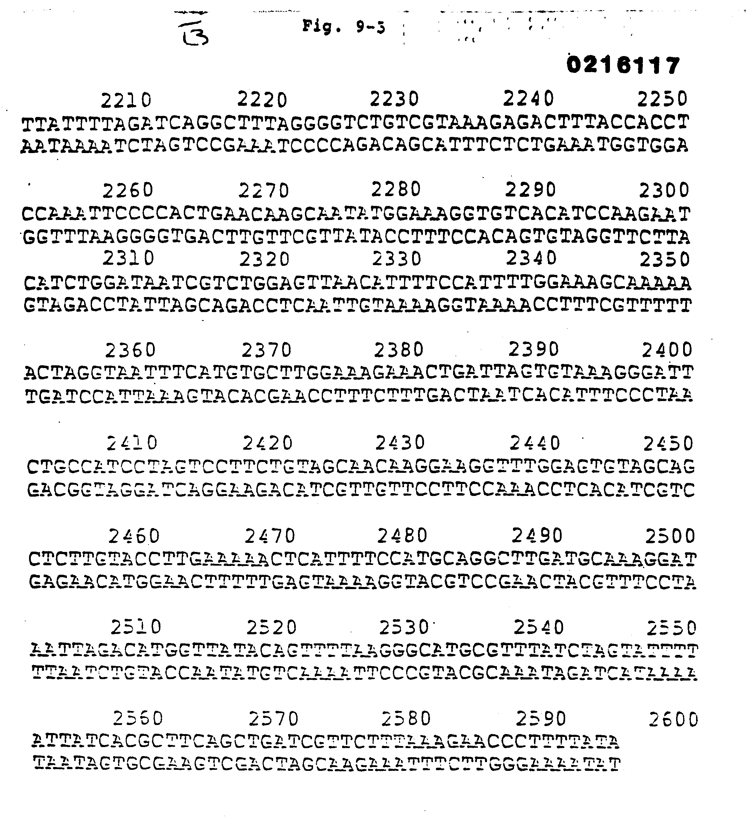

The present invention provides a DNA having a promoter activity of PHO81 gene regulating the production of phosphatase, and being obtainable from Saccharomyces cerevisiae; a DNA of the above-mentioned type and being a recombinant DNA; a DNA bearing a structural gene coding for a positive regulatory factor for production of repressible phosphatases or other structural gene at a position downstream from the PHO81 promoter; a transformant containing a DNA having a promoter activity of PHO81 gene regulating the production of phosphatase, and being obtainable from Saccharomyces cerevisiae; a transformant containing a DNA bearing the above PHO81 promoter and a structural gene downstream from the PHO81 promoter; and a process for the production of a gene product including the steps of cultivating in a culture medium a transformant containing a DNA having a promoter activity of PHO81 gene regulating the production of phosphatase, and being obtainable from Saccharomyces cerevisiae and a structural gene

0216117

located downstream from said PHO81 promoter, so that the transformant grows with the accumulation of the gene product, and recovering the gene product from the culture. The above-described DNA having a promoter activity of the PHO81 gene, is preferably, of which transcription is regulated with phosphoric acid. The above-described DNA having a promoter activity of the PHO81 gene, is preferably, a DNA bearing a base sequence of between the position 1 and cleavage site with the restriction enzyme SmaI(CCCGGG) in Fig.9;
GGGCCC

Detailed Description of the Invention:

A DNA according to the present invention containing the PHO81 promoter and a structural gene coding for the positive regulatory factor for the repressible phosphatase production (the PHO81 structural gene) may be isolated and collected from yeast cells.

Any strains of Saccharomyces cerevisiae may be used for this purpose. Especially suitable yeast strain is a strain of microorganism belonging to the genus Saccharomyces, such as S. cerevisiae. Commercially available baker's yeasts and brewer's yeast are particular examples of suitable yeast.

The extraction of DNA from yeast may be effected, for example, in accordance with the method described in Methods in Cell Biology, 12, 13-44 (1975).

The extracted DNA is treated with a suitable restriction enzyme to obtain a DNA fragment. The fragment is ligated with a plasmid or phage which has been digested with the same restriction enzyme as above or with a restriction enzyme capable of forming the same cohesive ends as those with the above enzyme, thereby to obtain a gene bank. The plasmid may be, for example, pBR322 or $\underline{E}$. $\underline{coli}$-yeast shuttle vector YEp13 [Gene, $\underline{8}$, 121 (1979)]. The phage may be, for example, charon phage [J. Virol., $\underline{29}$, 555 (1979)]. If necessary, sub-cloning may be further effected with the use of, for example, $\underline{E}$. $\underline{coli}$-yeast shuttle vector YEp6 [Gene, $\underline{8}$, 17 (1979)].

A host organism is then transformed with the vector bearing the thus cloned DNA. The host organism is preferably yeast, particularly a strain belonging to the genus $\underline{Saccharomyces}$, preferably a strain belonging to the species $\underline{Saccaromyces}$ $\underline{cerevisiae}$, more particularly a strain of $\underline{Saccharomyces}$ $\underline{cerevisiae}$ NA95-4B. It is preferred that the host organism be a pho81 mutant.

The strain NA95-4B may be obtained by customarily employed crossing method [Handbook of Genetics, p366, Plenum Press, New York (1974)]. That is, the strain NA95-4B may be obtained by crossing the strain AL211-12B (MAT$_\alpha$, pho3-1, pho8, arg6) [Mol. Cell. Biol., $\underline{2}$, 127 (1982)], the strain AH22 (MATa, leu2, his4, canl) [Proc. Natl. Acad. Sci. USA, $\underline{80}$, 1 (1983)], the strain D13-1A (MATa, trpl, his3, gal2, SUC2) [Proc. Natl. Acad. Sci. USA, $\underline{76}$, 1035 (1979)], the strain YAT228 (MATa, leu2, lysl0, cyh, karl-1) [J. Bacteriol., $\underline{145}$, 1421 (1981)] and the strain W755-1C (MATa, pho81, leu2, his3, his4).

The pho 81 mutant is transformed with the DNA of the gene bank constructed as described above or sub-clone to obtain a plasmid containing the PHO81 structure gene bearing the PHO81 promoter and open reading frame coding for regulatory factor of the repressible phosphatase production.

The transformation may be performed in any known manner, for example, in accordance with one of the methods described in Proc. Natl. Acad. Sci. USA, 75, 1929 (1978), Nature, 275, 104 (1978), Cold Spring Harbor Symp., Quant. Biol., 43, 1305 (1979), and Proc. Natl. Acad. Sci. USA, 76 1035 (1979), or with the similar methods.

Whether or not the transformant contains the PHO81 promoter and the coding region of the PHO81 gene producing the regulatory factor for the repressible phosphatases can be determined in the manner as follows.

The transformant is cultured in Rubin's modified medium (C. M. Rubin, low phosphoric acid complete medium) [Eur. J. Biochem., 41, 197 (1974)] to allow the formation of colonies. Then, the medium is overlaid with an agar layer containing α-naphthyl phosphate and fast blue salt B.

If the DNA containing the PHO81 promoter and the coding region of PHO81 protein, the regulatory factor for the repressible phosphatases, is cloned, the colonies will turn red, indicating the presence of the PHO81 gene. The plasmid is extracted from the transformant harboring the PHO81 gene and then digested with, for example, a restriction enzyme.

The digest is then subjected to agarose gel electrophoresis or acrylamide gel electrophoresis to fractionate a DNA fragment having the inserted gene. A series of these operations is well known in the art and described in detail in, for example, Molecular Cloning (1982), Cold Spring Harbor Laboratory.

The nucleotide sequence of the DNA containing the PHO81 gene may be determined by, for example, the dideoxynucleotide synthetic chain termination method [Proc. Natl. Acad. Sci. USA, 74, 5463 (1977)], Maxam-Gilbert method [Proc. Natl. Acad. Sci. USA, 74, 560 (1970)] and so on.

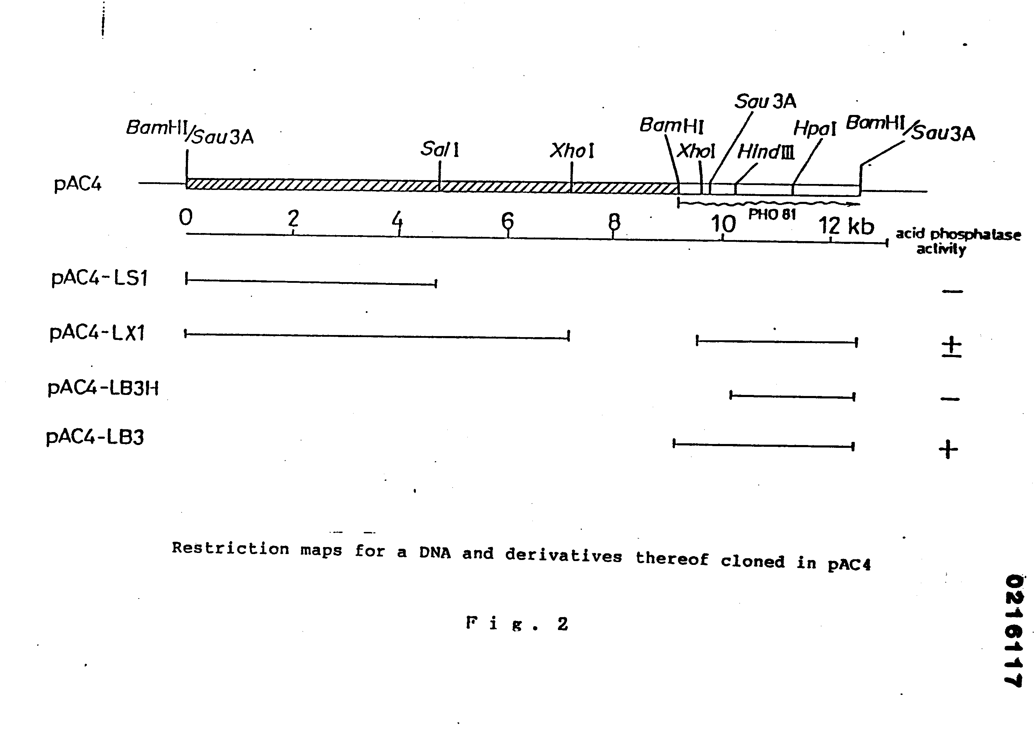

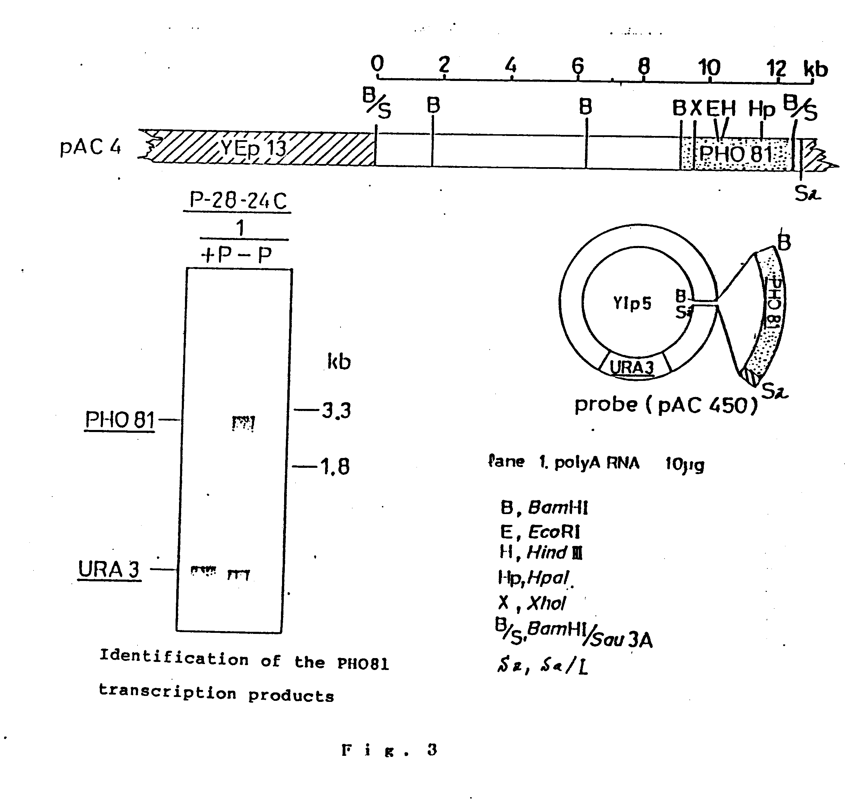

The position of the protein-coding region of the PHO81 gene may be deduced by examining the relationship between deleted plasmids and their complementation ability of the pho81 mutation(Fig. 2). Using the plasmid bearing the suspected PHO81 gene shown by white box in Fig. 2 as probe, a PHO81 transcript is detected. From the size of the transcript and the base sequence of PHO81 gene the position of the PHO81 promoter is estimated (Figs.3, 5 and 9).

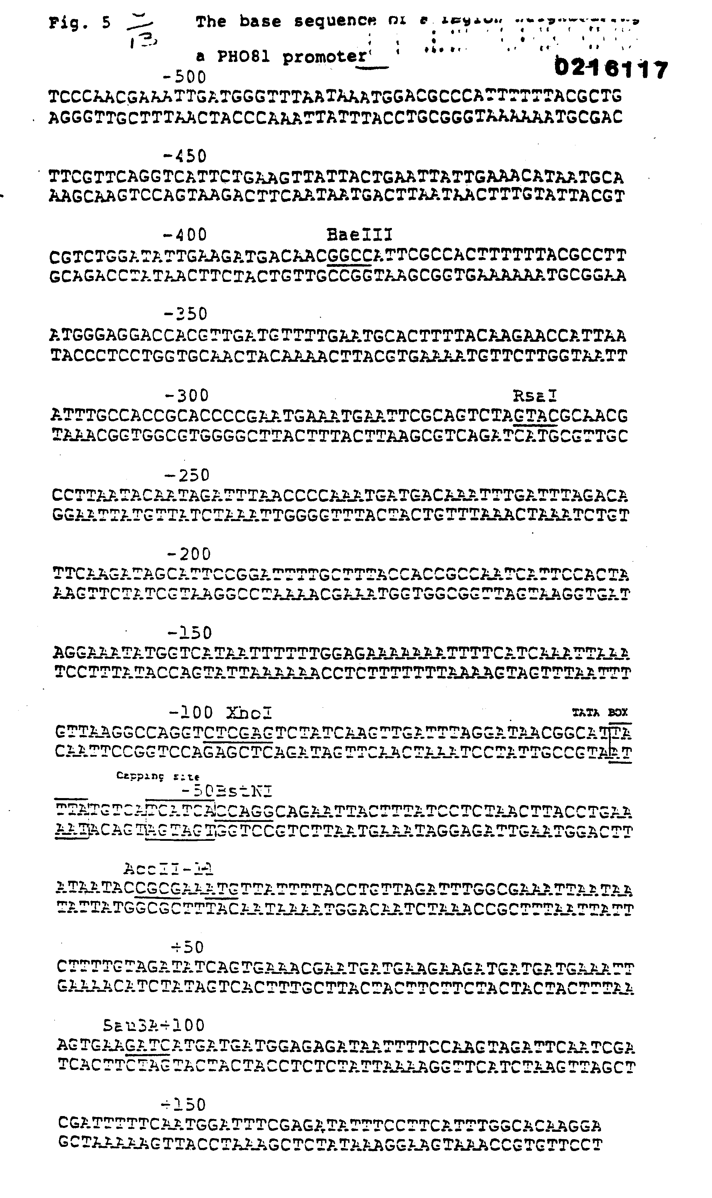

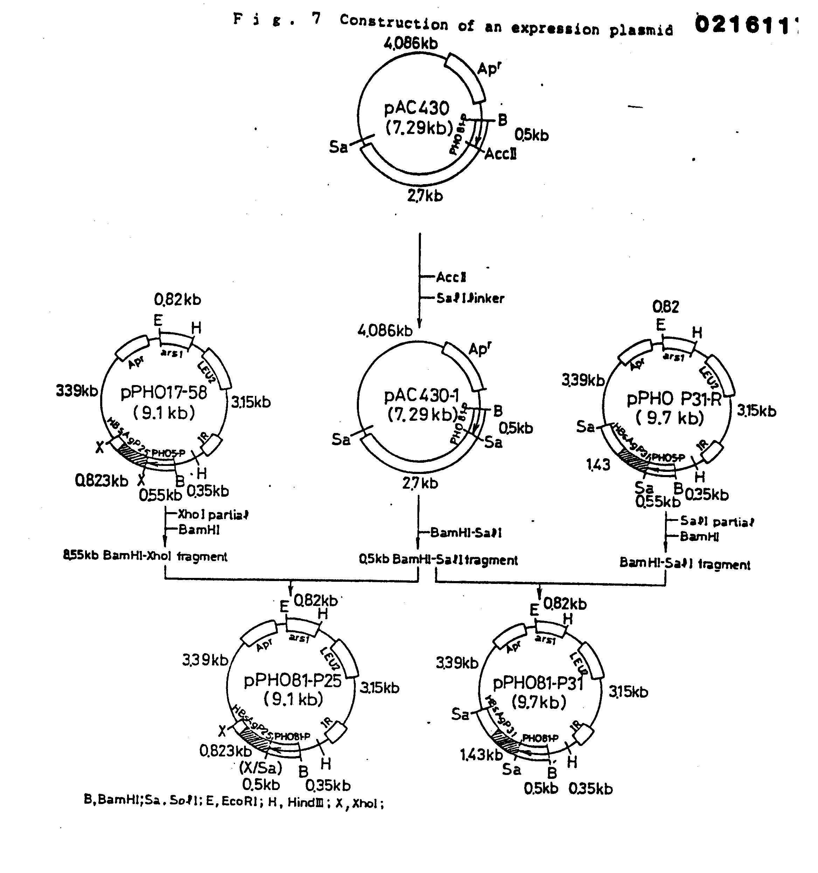

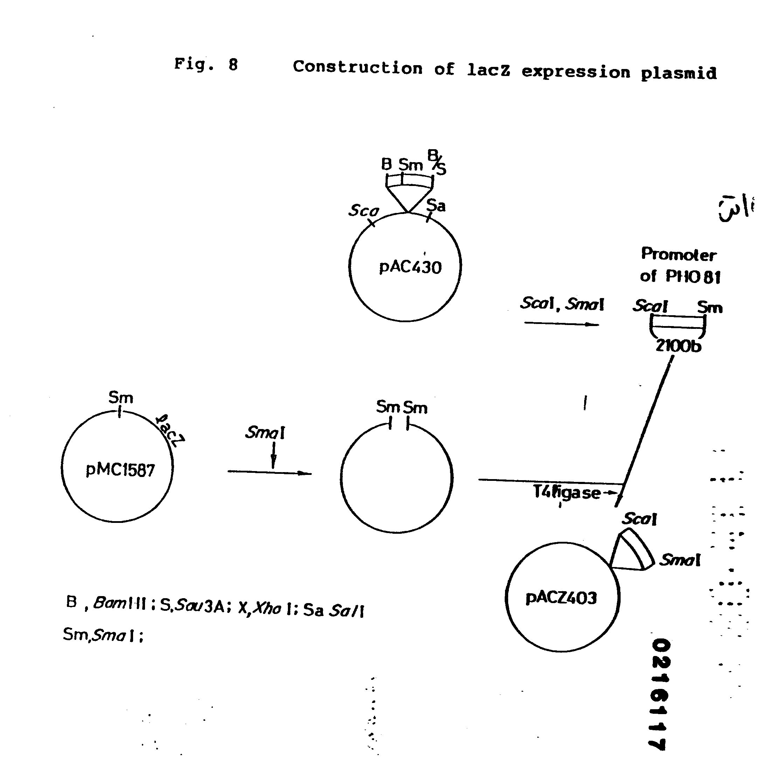

The base sequence of a region which is considered to contain the PHO81 promoter is then determined (Fig. 5). Determination of the nucleotide sequence indicates the presence of an open reading frame. The promoter is thus expected to locate upstream from the open reading frame. The portion of the DNA upstream from the open reading frame and having PHO81 promoter activity [ability to produce the regulatory factor for the repressible phosphatases production (See Table 1)] is prepared (Fig. 7). An expression vector is then prepared by inserting the portion having the PHO81 promoter activity and, if necessary, a desired structural gene downstream thereof into a vector (Fig. 8). The expression vector is inserted into a suitable host microbe which, upon culturing, produces the desired gene product.

The DNA bearing the PHO81 promoter activity may be entirely or partially synthesized by chemical processes and the synthetic or semisynthetic DNA may be used for the purpose of the present invention.

The vector into which the PHO81 promoter is inserted may be, for example, previously described shuttle vector YEp6 or YEp13 or plasmid pSH19 [Mol. Cell. Biol., 4, 771 (1984)] or pJDB219 [Nature, 275, 104 (1978)].

Illustrative of suitable structural genes to be inserted downstream from the PHO81 promoter are the regulating gene encoding the repressible acid phosphatase expression regulating factor (PHO81), the adw-type or adr-type hepatitis B virus surface antigen gene (HBsAg), human α-interferon gene, human β -interferon gene, human γ-interferon gene, human lisozyme gene, human interleukin-2 gene.

The host organism to be transformed with the PHO81 promoter-harboring DNA is preferably yeast, particularly a strain belonging to the genus Saccaromyces, preferably a strain belonging to the species S. cerevisiae, more particularly strains such as S. cerevisiae AH22R⁻ [Proc. Natl.

Acad. Sci. USA, 80, 1 (1983)] or S. cerevisiae NA95-4B (vide supra).

The transformants thus obtained may be cultured in any known medium such as Burkholder minimum medium [Proc. Natl. Acad. Sci. USA, 77, 4505 (1980)].

The culture conditions such as temperature and time may be determined so as to obtain the maximum yield of the desired gene product. Generally, a temperature of about 15-40°C, preferably about 24-37°C and a time of about 10-96 hours, preferably 24-72 hours are used. Aeration and agitation may be adopted, as necessary.

The gene product accumulated in the culture may be extracted in any known manner, such as by lyzing or disrupting the cells with the use of lysozyme such as Zymolyase (Seikagaku Kogyo, Ltd. Japan) or by meachanical disrupting method using glass beads. A detergent such as Triton-X100 and a protein denaturating agent such as guanidine hydrochloride may be used to facilitate the extraction of the product. The extract is then subjected to isolation and purification treatments conducted in conventional manner such as by precipitation using a precipitating agent, dialysis, electrophoresis, chromatography using ion exchange resins, gel filtration or a method using an antibody column.

In accordance with the present invention, there is provided a novel and potent promoter obtained from yeasts which are eukaryotic microorganisms. The promoter is very

0216117

useful for the effective expression of pharmacologically important protein gene.

The following examples will further illustrate the present invention.

Sacchromyces cerevisiae P-28-24C used as a starting material in Example 1 is deposited at the Institute for Fermentation, Osaka, Japan under the accession number of IFO-10153, and deposited at the American Type Culture Collection (ATCC), U.S.A., under the accession number of ATCC 60202. The transformant S. cerevisiae P-28-24C is deposited at IFO and ATCC under permanent deposition and freely available to any requester.

The transformant, Escherichia coli DHl/pAC430 shown in Example 8 is deposited at the Institute for Fermentation, Osaka, Japan under the accession number of IFO-14456 and has also been deposited at Fermentation Research Institute, Agency of Industrial Science and Technology, Ministry of International Trade and Industry, Japan (FRI) under the accession number of FERM P-8411 since August 9, 1985 and changed to the deposite under the accession number of FERM BP-1089 according to the Budapest Treaty.

The transformant, Saccharomyces cerevisiae AH22R⁻/pACZ403 shown in Example 12 is deposited at the Institute for Fermentation, Osaka, Japan under the accession number of IFO-10207 since May 28, 1986 and also deposited at FRI under the accession number of FERM BP-1090 according to the Budapest Treaty since June 25, 1986.

Example 1

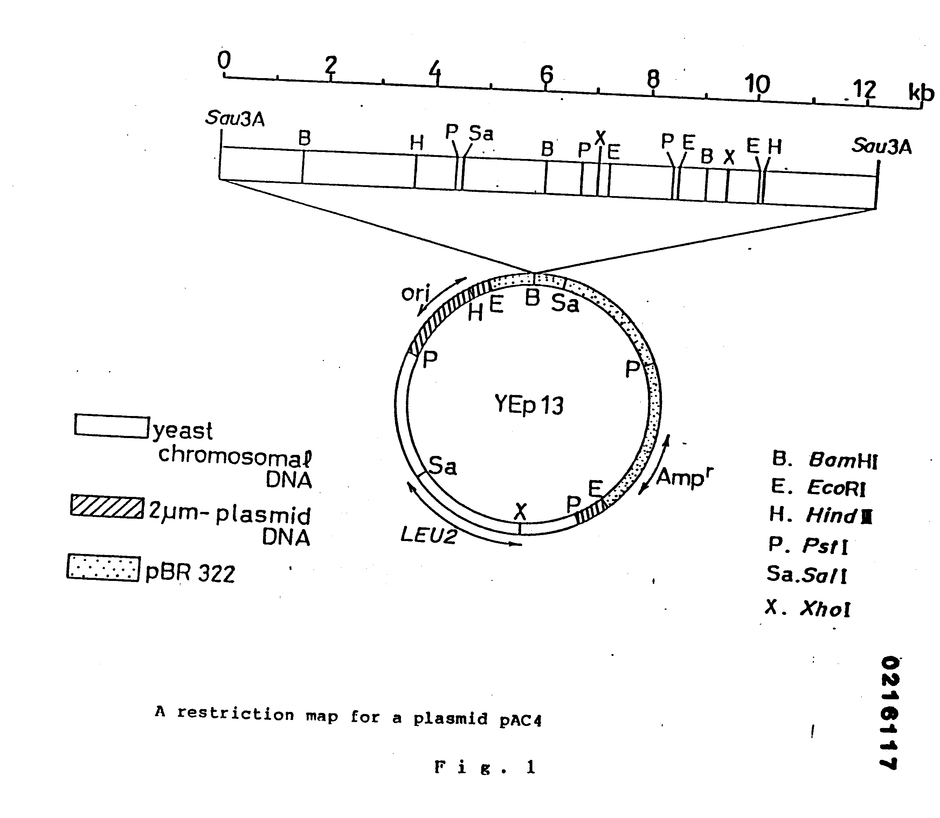

Cloning of the PHO81 gene (See Fig. 1):

Chromosomal DNA obtained in a conventional manner from S. cerevisiae P-28-24C (MATa pho3-1) (IFO 10153, ATCC 60202) was partially digested with Sau3AI. The Sau3AI restriction fragments were inserted into the BamHI site of yeast-E. coli shuttle vector YEp13 [J. R. Broach et al., Gene, 8, 121 (1979)] according to the method of Nasmyth and Reed [K. A. Nasmyth & S. I. Reed, Proc. Natl. Acad. Sci. USA, 77, 2119 (1980)] to prepare a yeast gene bank consisting of about 2000 clones of E. coli showing the ampicillin resistant (Amp$^r$) and tetracycline sensitive (Tc$^s$) phenotype. The plasmid DNA of the yeast gene bank was introduced into the strain S. cerevisiae NA95-4B (MATα pho81 leu2 his3 trpl canl) for transformation. Transformants showing repressible acid phosphatase producing activity (rACp$^+$) were screened by the colony staining method [modified method of G. Dorn, Genet. Res., 6, (1965)] using 1 % agar solution containing 0.5 mg/ml of α-naphthylphosphate, 5 mg/ml of fast blue salt B and 0.05 M acetate buffer. Five rACp$^+$ transformants were obtained from about $8 \times 10^3$ transformants which are prototrophic for leucine (Leu$^+$). One of the five rACp$^+$ transformants was used to prepare a plasmid DNA according to the method of Cameron et al [J. R. Cameron et al., Nucleic Acids Res., 4, 1429 (1977)] and the plasmid DNA was transformed into E. coli JA221. All of the 35 Amp$^r$ transformants were Tc$^s$ and had a plasmid of the same molecular weight.  This plasmid was named pAC4 and was again introduced into S. cerevisiae NA95-4B for transformation.  Eighteen of the thus obtained Leu$^+$ transformants were tested for their repressible acid phosphatase production activity to reveal that all of them were rACp$^+$.  This suggests that pAC4 has an insertion of a DNA fragment bearing the PHO81 gene.  If PHO81 is included, then pAC4 is expected to be able to be integrated into the pho81 locus of chromosome by virtue of its homology.  A stable transformant of S. cerevisiae YAT408 (leu2, lys10, can1, cir$^0$) containing pAC4 was subjected to genetic analysis for the determination of the integraion site of pAC4.  Thus, the transformant containing pAC4 was crossed with S. cerevisiae YAT637 (MATa pho81 leu2) and was subjected to a tetrad analysis.  As a result, the segregation of Leu$^+$Acp$^+$ and Leu$^-$Acp$^-$ phonotypes in tetrad showed 4:0, 3:1 and 2:2.  The numbers of tetrads showing 4:0, 3:1 and 2:2 segregations were 0, 2 and 21, respectively.  A second cross was cerried out with the pAC4-containing transformant and S. cerevisiae YAT61 (MATa PHO81 LEU2).  The tetrad analysis of the resultant diploid revealed that a ratio of the tetrads showing 4+:0-, 3+:1- and 2+:2- segregations of Leu phenotype was 2:33:4.  The foregoing genetic data permit one to conclude that pAC4 is inserted on PHO81 gene site or its vicinity, i. e. the PHO81 gene is cloned into pAC4.

## Example 2

### Preparation of Restriction Enzyme Map for
### DNA Fragment Bearing the PHO81 Gene:

The pAC4 DNA (1-5 µg) was digested in TA buffer [P. H. O'Farrell et al., Molec. Gen. Genet., 179, 421 (1980)] with 4-6 units of single or a combination of restriction enzymes selected from BamHI, EscoRI, HindIII, PstI, SalI and XhoI at 37°C for 1 hour. The digestion products were electrophoresed on a 1% agarose gels or 7.5% polyacrylamide gel and the gels were examined for the restriction patterns to estimate molecular weights of the restriction fragments. Then, a restriction enzyme cleavage map was prepared as shown in Fig. 1, in which restriction sites are designated by letters as follows:

| | | | |
|---|---|---|---|
| E: | EcoRI | H: | HindIII |
| Sa: | SalI | X: | XhoI |
| P: | PstI | B: | BamHI |

## Example 3

### Estimation of the Position of PHO81 Gene on
### Cloned DNA Fragment (See Fig. 2):

In order to estimate the position of the PHO81 gene in the cloned DNA fragment, various deletion derivatives of pAC4 were prepared. Thus, pAC4 (5 µg) was digested in 50 µl of TA buffer containing 6 units of BamHI at 37°C for 1 min and then at 65°C for 10 min to obtain a partially digested product. A 25 µl-portion of the digestion mixture was mixed with 50 µl of T4 ligase solution containing 5 mM MgCl$_2$, 10 mM dithiothreitol, 0.05 mM ATP and 3 units of T4 ligase and the mixture was stand at 4$^{\circ}$C for 18 hours to religate the partially digested product described above. E. coli JA221 was transformed with the use of the T4 ligase reaction solution (10 µl). From the Amp$^r$ transformants thus obtained, a plasmid DNA was isolated in accordance with the method of Birnboim and Doly [H. C. Birnboim & J. Doly, Nucleic Acids Res., 7, 1513 (1979)], thereby to obtain deleted plasmid pAC4-LB3. The above procedures were repeated in the same manner as described using SalI and XhoI in place of BamHI to obtain pAC4-LS1 and pAC4-LX1, respectively. Further, pAC4-LB3 was treated in the same manner as above using HindIII to obtain deleted plasmid pAC4-LB3H. Each of the resultant deleted plasmid DNA was used for transformation of S. cerevisiae NA95-4B and Leu$^+$ transformants were selected. Ten such transformant strains were tested for their phenotype with respect to repressible acid phosphatase (rACp$^+$l). The result of complementation tests of the pho 81 mutation with the deletion plasmids is shown in Fig.2 in terms of + and -.

S. cerevisiae NA95-4B was transformed with pAC4-LX1. The rACp producing activity of the resultant Leu$^+$ transformants is lower than that of the wild type strain (the activity is shown as + in Fig. 2). The foregoing results indicate that the PHO81 gene is located at a region (about 3.0 kb) shown by white box in Fig. 2.

Example 4

## Acid Phosphatase Producing Activity

## of Transformant Containing pAC4-LB3:

The pAC4-LB3-containing transformant S. cerevisiae NA95-4B/pAC4-LB3 obtained in Example 3 was cultivated in 5 ml of Berkholder's modified high phosphoric acid medium and low phosphoric acid medium [A. Toh-e et al, J. Bacteriol., 113, 727 (1973)] for 20 hours with shaking. The cells were collected for measuring the acid phosphatase activity in accordance with the modified method of Torriani [A. Torriani, Biochim. Biophys. Acta, 38, 460 (1960)]. The results were as shown in Table 1.

As will be understood from Table 1, the production of the acid phosphatase by pAC4-LB3 is repressed by inorganic phosphate. Thus, the cloned PHO81 gene is considered to include both a region coding for the protein (PHO81 gene product) which regulates the expression of PHO5 and a promoter region.

Table 1

Acid Phosphatase Production Activity

of Transformants Containing pAC4-LB3

| Strain of S. cerevisiae | Genotype [a] | Acid Phosphatase Activity* | |
|---|---|---|---|
| | | High Phosphoric Acid [b] | Low Phosphoric Acid [c] |
| P-28-24C | Wild type | 0.003 | 0.099 |
| NA95-4B/ pAC4-LB3 | pho81[pAC4-LB3] | 0.003 | 0.102 |
| NA95-4B | pho81 | 0.004 | 0.005 |

a) Indicated is only with respect to phosphatase

\* Units/ml/OD$_{660}$ —

b) $KH_2PO_4$ concentration of 1.5 mg/ml

c) $KH_2PO_4$ concentration of 0.03 mg/ml

Example 5

Identification of PHO81 Gene Transcript:

The transcription product of PHO81 was identified
according to the Northern hybridization method. Total RNA was
prepared from S. cerevisiae P-28-24C cultured in a complete
medium (+P) and a low phosphoric acid medium (-P) in
accordance with the method of Jensen [R. jensen et al., Proc.
Natl. Acad. Sci. USA, 80, 3035 (1983)], followed by
purification by affinity column chromatography on oligo
dT-cellulose in accordance with the method of Schleif and
Wensink [R. F. Schleif & P. C. Wensink, Practical Methods in
Molecular Biology, (1981), Springer-Verlag] to obtain
poly(A)$^+$RNA. The poly(A)$^+$RNA sample thus obtained was
subjected to formaldehyde gel electrophoresis as described in
the article [Molecular Cloning, (1982), Cold Spring Harbor
Laboratory], followed by blotting and hybridization according
to the method of Thomas [P. S. Thomas, Proc. Natl. Acad. Sci.
USA, 77, 5201 (1980)]. Autoradiography was performed at -80$^{\circ}$C
employing a Kodak X-O-mat RP film and a Kodak intensifying
screen. The probe DNA used for the identification of the
transcript was prepared by nick translation [P. W. J. Rigby et
al., J. Mol. Biol., 113, 237 (1977)] of a plasmid pAC450 obtained by subcloning a BamHI-SalI restriction fragment of about 3.2 kb, which was located between the BamHI restriction cleavage site in the vicinity of 9.2 kb of the cloned yeast chromosomal DNA (Fig. 1) and the SalI restriction cleavage site of YEp13, into pBR322 double-digested with BamHI and SalI.

The results are shown in Fig. 3. The size of the PHO81 transcript is 2.8 kb. The fact that the amount of the PHO81 transcript is much greater than that of URA3 gene which codes for orotidine-5'-phosphate-decarboxylase [The Molecular Biology of the Yeast Saccharomyces, life cycle and inheritance, Cold Spring Harbor Lab., 731 (1981)] suggests the strong activity of the PHO81 promoter. Since the transcription of PHO81 is repressed in the high phosphate environment (+P), the PHO81 promoter is considered to be repressed by phosphoric acid.

Example 6

Preparation of Restriction Enzyme Map for 3.0 kb
DNA Fragment Bearing the PHO81 Gene:

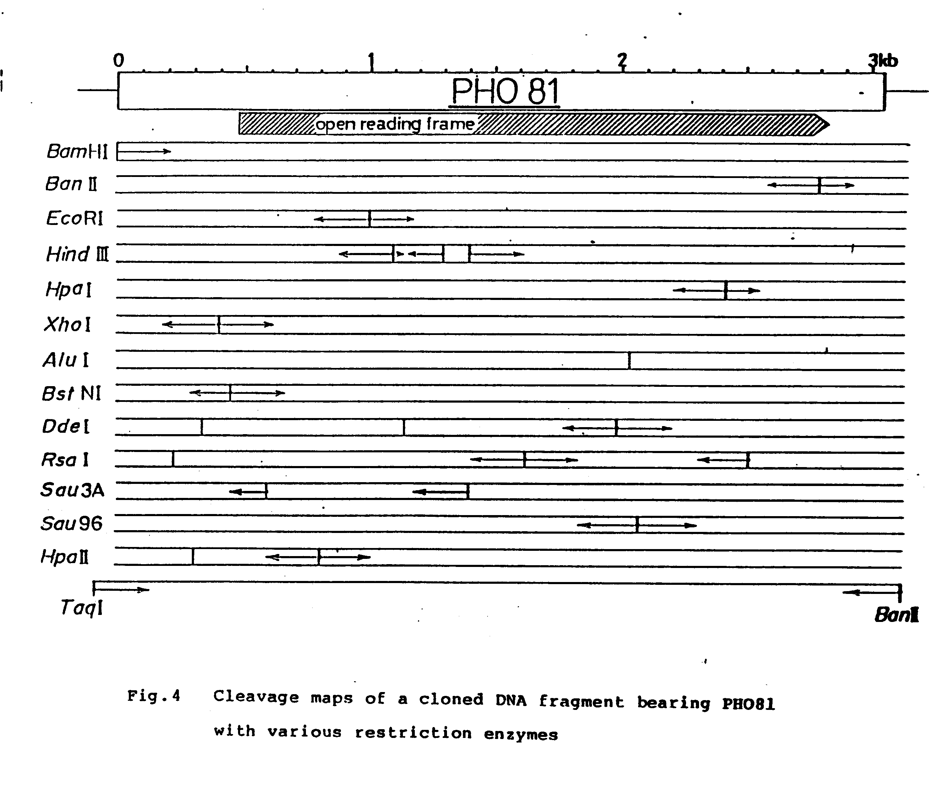

The restriction enzyme map for the restriction fragment between the BamHI site and the Sau3AI/BamHI site including the PHO81 gene (the region shown by white box in Fig. 2) was prepared with the use of 14 kinds of restriction enzymes (AluI, BanII, BstNI, DdeI, EcoRI, HaeIII, HindIII, HpaI, HpaII, RsaI, SalI, Sau3AI, Sau96I and XhoI) and is shown in Fig. 4. The fragment was digested with one or a combination of two of the enzymes.  The digestion mixtures were electrophoresed on 7.5% or 12% polyacrylamide gels. Restriction enzyme cleavage sites were estimated from the cleavage patterns.  A HaeIII or AluI digest of pBR322 was used as a molecular weight marker.  Only the restriction enzyme cleavage sites whose positions are confirmed are shown in Fig. 4.

Example 7

Determination of the Base Sequence of DNA

Bearing the PHO81 Gene:

The base sequence of the DNA fragment of 3.0 kb located between BamHI and BamHI/Sau3A sites (See Fig. 2) and considered to bear the PHO81 gene was determined according to the method of Maxam and Gilbert (vide supra) and is shown in Fig. 5.  In the nucleotide sequence, there exists a translatable region of about 2.5 kb.  From this frame, the direction of transcription is expected to be from the BamHI site to the BamHI/Sau3A site.  A translational initiation codon "ATG" is present at about 520 bases downstream from the BamHI cleavage site.  At 67 bp (base pairs) upstream from the "ATG", there is present a sequence TATTA which is considered to function as a TATA box.  A sequence TCATCA, which is similar to capping site, exists at 57 bp upstream from the "ATG".  Further, there is a sequence CCAAT at 109 bp upstream from the "ATG".  This sequence is identical with that of CCAAT box [Nucleic Acids Res., 10, 2625-2637 (1982); ibid 12, 857-872 (1984); ibid 12, 1137-1148 (1984)]. Fig. 5 shows the base sequence of a region of 700 bases downstream from the BamHI cleavage site. A non-translational region of 5'-upstream side of the PHO81 gene and a 5'-terminal region of the PHO81 gene are considered to be included with in the illustrated region.

Example 8

### Construction of Plasmid pAC430 and Preparation of Transformant Using Same

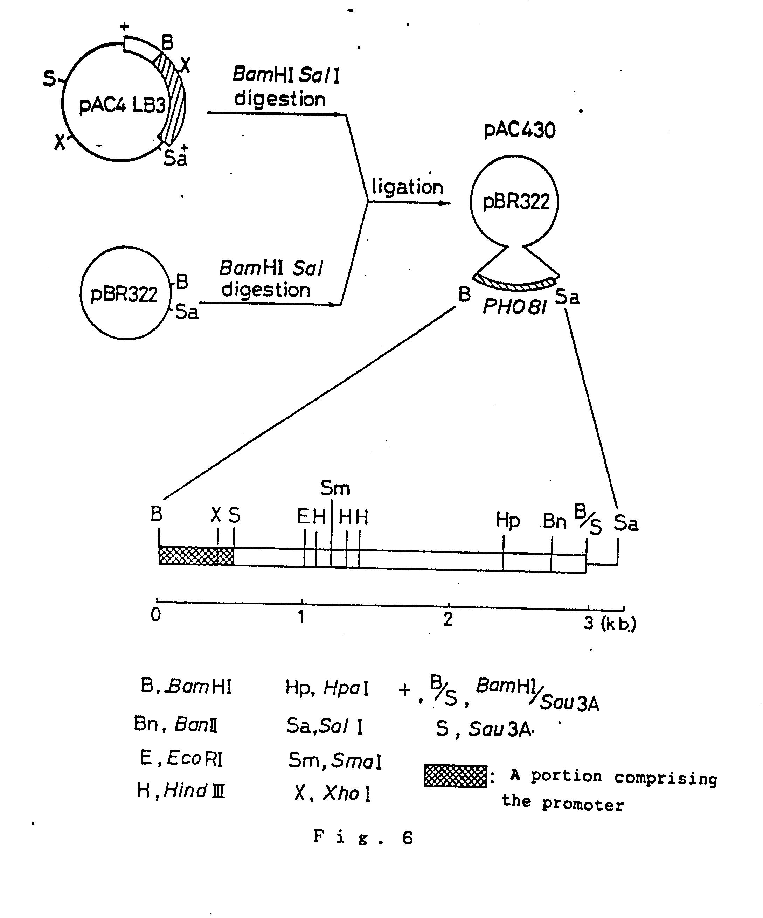

The plasmid pAC4-LB3 (5 µg) was digested with 5 units of BamHI and 5 units of SalI in the manner described in Example 2. The digestion mixture was subjected to electrophoresis on a 1% low melting agarose gel to recover a DNA fragment of 3.2 kb. This fragment was mixed with pBR322 (5 µg) digested with 5 unites of BamHI and 5 units of SalI, and the mixture was ligated by T4 ligase in the same manner as described in Example 2 to obtain a plasmid pAC430 (See Fig. 6). The plasmid pAC430 was introduced into E. coli DH1 for transformation to obtain a transformant Escherichia coli DH1/pAC430(IFO 14456, FERM BP-1089).

Example 9

### Construction of adw-Type Hepatitis B Virus Surface Antigen P25 Gene Expression Plasmid Using the PHO81 Promoter and Transformation of Yeast with the Plasmid

The plasmid pAC430 (0.5 µg) is digested with 1 unit of

0216117

restriction enzyme AccII (manufactured by Nippon Gene Inc.) in 20 μl of a reaction medium [6mM Tris-HCl (pH7.5), 60 mM NaCl, 6 mM $MgCl_2$, 6 mM 2-mercaptoethanol] at $37^O$C for 2 hour, followed by elimination of protein with phenol and the DNAs were precipitated by addition of cold ethanol. The precipitated DNA is mixed with 50 ng of a SalI linker having a phosphorylated 5'-terminal [5'-P-d(GGTCGACC)] (manufactured by New England Biolabs Inc.) and the mixture was reacted in 20 μl of a reaction liquid containing 66 mM Tris-HCl (pH7.6), 6.6 mM $MgCl_2$, 10 mM dithiothreitol, 1 mM ATP and 2 units of T4 DNA ligase (manufactured by New England Biolabs. Inc.) at $14^O$C overnight to ligate the DNA. E. coli DH1 [T. Maniatis et al., Molecular Cloning, Cold Spring Harbor Laboratory, 254-255 (1982)] is transformed with the use of the above ligation liquid, and ampicillin resistant transformants were selected. From the selected transformants, plasmid pAC430-1 having a SalI site substituted for the AccII site of the plasmid pAC430 is obtained (See Fig. 7). The plasmid pAC430-1 (10 μg) is digested with 10 units of restriction enzyme BamHI and 10 units of restriction enzyme SalI (manufactured by Nippon Gene Inc.) in 50 μl of a reaction medium [10 mM Tris-HCl (pH7.5), 7 mM $MgCl_2$, 100 mM NaCl, 7 mM 2-mercaptoethanol] at $37^O$C for 2 hours. The digest is then applied to 1.2% agarose-slab gel and electrophoresed in a buffer [100 mM Tris-HCl, 100 mM boric acid, 2 mM EDTA (pH8.3)] at 140 V for 2 hours. After the electrophoresis, the region of the gel containing 0.5 kb DNA fragment is placed in a dialysis tube and immersed in the above electrophoresis buffer.  The DNA fragment is extracted from the gel by electrical elution [M. W. McDonell et al., J. Mol. Biol., 110, 119 (1977)].  The liquid within the dialysis tube is extracted with phenol and then with ether.  Following extraction, the aqueous phase is adjusted to 0.2 M with NaCl. The DNA fragment containing the PHO81 promoter is precipitated by the addition of 2 volumes of cold ethanol.

The adw-type hepatitis B virus surface antigen (HBsAg P25) gene expression plasmid pPHO17-58 (50 μg) disclosed in Example of the specification of Japanese Patent Application No.59-193765 filed September 13, 1984 (which corresponds to European Patent Publication No.175283) is partially digested with 50 units of restriction enzyme XhoI (manufactured by Nippon Gene Inc.) in 100 μl of a reaction medium [10 mM Tris-HCl (pH 7.5), 7 mM $MgCl_2$, 100 mM NaCl, 7 mM 2-mercaptoethanol] at $37^{\circ}C$ for 20 min.  From the digest mixture is separated a DNA fragment of 9.1 kb, which is cleaved at only one of the two XhoI restriction enzyme cleavage sites of the plasmid, by means of the agarose-slab gel under the same condition as described previously.

The DNA fragment of 9.1 kb (4 μg) thus recovered is then digested with 4 units of restriction enzyme BamHI in 20 μl of a reaction medium [6 mM Tris-HCl (pH7.9), 150 mM NaCl, 6 mM $MgCl_2$] at $37^{\circ}C$ for 2 hours, followed by electrophoresis conducted under the same conditions as above, thereby to isolate a 8.55 kb DNA.

The 8.55 kb DNA (200 ng) is ligated with the 0.5 kb DNA (20 ng) containing the PHO81 promoter using T4 DNA ligase. With the use of the resultant mixture, E. coli DH1 is transformed. From the ampicillin resistant transformants, transformant DH1/pPHO81-P25 containing the plasmid pPHO81-P25 in which the PHO81 promoter is inserted in the sense direction as the HBsAgP25 gene is selected. Plasmid pPHO81-P25 is then isolated from the transformant (See Fig. 7) and introduced into a yeast strain of Saccharomyces cerevisiae AH22R⁻, thereby to obtain transformant AH22R⁻/ pPHO81-P25.

Example 10

Construction of an Expression plasmid for the production of adr-type Hepatitis B Virus Surface Antigen P31 Using PHO81 Promoter and Transformation of Yeast with the Plasmid

The adr-type hepatitis B Virus surface antigen (HBsAg P31) gene expression plasmid pPHO P31-R (50 μg) disclosed in Example 3 of International Patent Application No. PCT/JP84/423 (International Filing Date: September 4, 1984) (which corresponds to European Patent Publication No.171908) is digested with 50 units of restriction enzyme SalI in 100μl of a reaction medium [6 mM Tris-HCl (pH 7.9), 150 mM NaCl, 6 mM MgCl$_2$, 6 mM 2-mercaptoethanol] at 37°C for 20 min. From the digest is separated a DNA fragment of 9.7 kb, which is cleaved at only one of the two SalI restriction enzyme cleavage sites of the plasmid, by means of the agarose-slab gel in the same manner as above.

The DNA fragment of 9.7 kb (4 µg) thus recovered is then digested with 4 units of restriction enzyme BamHI, followed by electrophoresis under the same conditions as above, thereby to isolate a 9.2 kb DNA. The 9.2 kb DNA (200 ng) is ligated with the 0.5 kb DNA (20 ng) containing the PHO81 promoter with the employment of T4 DNA ligase. Using the resultant mixture, E. coli DH1 is transformed. From the ampicillin resistant transformants, transformant DH1/pPHO81-P31 containing the plasmid pPHO81-P31 in which the PHO81 promoter is inserted in the right direction as the HBsAg P31 gene is selected. Plasmid pPHO81-P31 is then isolated from the transformant (See Fig. 7) and introduced into yeast host Saccharomyces cerevisiae AH22R⁻ to obtain transformant AH22R⁻/pPHO81-P31.

Example 11

Expression of the HBsAg Gene in Yeast:

The transformants Saccharomyces cerevisiae AH22R⁻/pPHO81-P25 and AH22R⁻/pPHO81-P31 containing the HBsAg gene expression plasmid and obtainable in Examples 9 and 10 are cultivated at 30°C for 2 days in Burkholder medium and its low phosphoric acid medium. Cells are collected and washed with physiological saline solution. The cells are then treated with Zymolyase (Seikagaku Kogyo Co. Ltd., Japan) in accordance with the method of Miyanohara [A. Miyanohara, Proc.

Natl. Acad. Sci. USA, <u>80</u>, 1 (1983)] to form spheroplasts.
After adding 0.1% of Triton X-100 for expediting extraction of
HBsAg, the lysate is centrifuged at 15,000 rpm for 15 min at
room temperature. The supernatant liquid was tested for the
detection of HBsAg activity by means of Orthzyme II
(manufactured by Dynabot K. K.).

<u>Example 12</u>

<u>Expression of lacZ Gene Using PHO81 Promoter</u>:

Using respective 10 units of restriction enzyme ScaI and
SmaI (manufactured by Takara Shuzo Co. Ltd., Japan ), 10 μg
of pAC430 containing PHO81 gene (the region from the white box
portion in Fig. 2 to the SalI site of YEp13) were digested in
100 μl of a reaction medium [33 mM Tris-acetic acid (pH7.9),
66mM K-acetate, 10mM Mg-acetate, 5mM dithiothreitol] at 37$^{o}$C
for 2 hours. The digest was applied to 4% acrylamide gel and
electrophoresed in buffer (89 mM Tris, 89 mM boric acid, 2.5
mM EDTA) at 150 V for 3 hours. Following the electrophoresis,
the portion of the gel containing 2100 bp DNA fragment
(ScaI-SmaI restriction fragment) was placed in a Corex tube
for the destruction thereof, to which was then added 5 ml of a
DNA extraction buffer of 0.5 M $(NH_4)COOCH_3$, 10 mM Mg-acetate,
1 mM EDTA, 0.1% (w/v) SDS. The resultant mixture was allowed
to stand overnight at 37$^{o}$C and filtered. The DNA fragment was
precipitated by the addition of ethanol to the filtrate and
recovered for use in the ligation reaction described below.

Plasmid pMC1587 (1 μg) was digested with 1 unit of restriction enzyme SmaI (manufactured by Takara Shuzo Co. Ltd.) in 50 μl of a reaction mixture (1 mM Tris, 10 mM NaCl, 0.6 mM MgCl$_2$) at 37$^{O}$C for 2 hours. The restriction DNA· fragment was precipitated with ethanol and recovered. The above described DNA fragment(0.1 μg) from pMC1587 digested with SmaI was ligated with the ScaI-SmaI restriction fragment of 2100 bp(5 μg) using T4 DNA ligase to produce a plasmid pACZ403(Fig. 8). In the thus obtained plasmid, a portion of the PHO81 translational sequence located in the ScaI-SmaI DNA fragment of 2100 bp was ligated at the SmaI connecting site with the lacZ translational sequence located in the pMC1587, with the reading frames of both genes being ajusted.

The thus obtained plasmid pACZ403 was introduced into _Saccharomyces_ _cerevisiae_ AH22R⁻ to obtain transformant _Saccharomyces_ _cerevisiae_ AH22R⁻/pACZ403(IFO-10207, FERM BP-1090). The transformant was cultured for 20 hours in the same manner as described in Example 11 and the cells were treated in the same manner as described in Example 11 to obtain the following results.

|  | The expression of β-galactosidase unit/l |
| --- | --- |
| The medium containing high concentration of phosphoric acid (KH$_2$PO$_4$ 1.5g/l) | 600 |
| The medium containing low concentration of phosphoric acid (KH$_2$PO$_4$ 0.3g/l) | · 2600 |

0216117

## Example 13

### Sequencing of PHO81 Gene:

The DNA sequence of the BamHI-BanII fragment(about 2.6 kb) containing the PHO81 gene was determined according to the method of Maxam and Gilbert described in Example 7 and is shown in Fig. 9.  In the base sequence, there exists a "stop codon" at about 2330 bp region.

What Is Claimed Is:

1. A DNA having a promoter activity of PHO81 gene regulating the production of phosphatase, and which is obtainable from Saccharomyces cerevisiae.

2. A DNA as recited in claim 1 wherein said PHO81 gene of which transcription is regulated with phosphoric acid.

3. A DNA as recited in claim 1 and bearing a base sequence of between the position 1 and cleavage site with restriction enzyme SmaI in Fig. 9.

4. A DNA as recited in claim 1, 2 or 3 and being a recombinant DNA.

5. A DNA as recited in claim 1, 2, 3 or 4 having a structural gene downstream from the PHO81 promoter.

6. A DNA as recited in claim 5, wherein the structural gene is one other than that coding for a positive regulatory factor for production of repressible phosphatases.

7. A DNA as recited in claim 6, wherein the structural gene is selected from the group consisting of the adw-type hepatitis B virus surface antigen P25 gene, the adr-type hepatitis B virus surface antigen P31 gene and the lacZ gene.

8. A transformant containing a DNA having a promoter activity of PHO81 gene regulating the production of phosphatase, and which is obtainable from Saccharomyces cerevisiae.

9. A transformant as recited in claim 8 wherein said PHO81 gene of which transcription is regulated with phosphoric acid.

10. A transformant as recited in claim 8 wherein the DNA bears a base sequence of between the position 1 and cleavage site with restriction enzyme SmaI in Fig. 9.

11. A transformant as recited in claim 8, 9 or 10 wherein the DNA has a structural gene downstream from the PHO81 promoter.

12. A transformant as recited in claim 8 and being Escherichia coli DH1/pAC430 (FERM BP-1089).

13. A transformant as recited in claim 8, wherein the host organism is yeast.

14. A transformant as recited in claim 8 and being Saccharomyces cerevisiae AH22R$^{-}$/pPHO81-P25.

15. A transformant as recited in claim 8 and being Saccharomyces cerevisiae AH22R⁻/pPHO81-P31.

16. A transformant as recited in claim 8 and being Saccharomyces cerevisiae AH22R⁻/pACZ403(FERM BP-1090).

17. A process for the production of a gene product, comprising the steps of:

culturing in a culture medium a transformant containing a DNA having a promoter activity of PHO81 gene regulating the production of phosphatase, and being obtainable from Saccharomyces cerevisiae, and a structural gene located downstream from said PHO81 promoter so that the transformant grows with the accumulation of the gene product; and

recovering the gene product from the culture.

18. A process as recited in claim 17 wherein said PHO81 gene of which transcription is regulated with phosphoric acid.

19. A process as recited in claim 17 the DNA bears a base sequence of between the position 1 and cleavage site with restriction enzyme SmaI in Fig. 9.

A restriction map for a plasmid pAC4

Fig. 1

Restriction maps for a DNA and derivatives thereof cloned in pAC4

F i g . 2

Identification of the PHO81 transcription products

lane 1. polyA RNA    10μg

B , *BamHI*
E , *EcoRI*
H , *Hind III*
Hp, *HpaI*
X , *XhoI*
B/S, *BamHI*/*Sau 3A*
Sℓ, *Sa/L*

F i g . 3

Fig.4    Cleavage maps of a cloned DNA fragment bearing PHO81
with various restriction enzymes

Fig. 5   The base sequence of a region comprising
a PHO81 promoter

```
        -500
TCCCAACGAAATTGATGGGTTTAATAAATGGACGCCCATTTTTTACGCTG
AGGGTTGCTTTAACTACCCAAATTATTTACCTGCGGGTAAAAATGCGAC


        -450
TTCGTTCAGGTCATTCTGAAGTTATTACTGAATTATTGAAACATAATGCA
AAGCAAGTCCAGTAAGACTTCAATAATGACTTAATAACTTTGTATTACGT


        -400            BaeIII
CGTCTGGATATTGAAGATGACAACGGCCATTCGCCACTTTTTTACGCCTT
GCAGACCTATAACTTCTACTGTTGCCGGTAAGCGGTGAAAAAATGCGGAA


        -350
ATGGGAGGACCACGTTGATGTTTTGAATGCACTTTTACAAGAACCATTAA
TACCCTCCTGGTGCAACTACAAAACTTACGTGAAAATGTTCTTGGTAATT


        -300                                   RsaI
ATTTGCCACCGCACCCCGAATGAAATGAATTCGCAGTCTAGTACGCAACG
TAAACGGTGGCGTGGGGCTTACTTTACTTAAGCGTCAGATCATGCGTTGC


        -250
CCTTAATACAATAGATTTAACCCCAAATGATGACAAATTTGATTTAGACA
GGAATTATGTTATCTAAATTGGGGCTTTACTACTGTTTAAACTAAATCTGT


        -200
TTCAAGATAGCATTCCGGATTTTGCTTTACCACCGCCAATCATTCCACTA
AAGTTCTATCGTAAGGCCTAAAACGAAATGGTGGCGGTTAGTAAGGTGAT


        -150
AGGAAATATGGTCATAATTTTTTGGAGAAAAAATTTTCATCAAATTAAA
TCCTTTATACCAGTATTAAAAAACCTCTTTTTTTAAAAGTAGTTTAATTT


        -100 XhoI                                TATA BOX
GTTAAGGCCAGGTCTCGACTCTATCAAGTTGATTTAGGATAACGGCATTA
CAATTCCGGTCCAGAGCTCAGATAGTTCAACTAAATCCTATTGCCGTAAT

    Capping site
        -50 BstNI
TTATGTCATCATCACCAGGCAGAATTACTTTATCCTCTAACTTACCTGAA
AATACAGTAGTAGTGGTCCGTCTTAATGAAATAGGAGATTGAATGGACTT


        AccII-14
ATAATACCGCGAAATGTTATTTTACCTGTTAGATTTGGCGAAATTAATAA
TATTATGGCGCTTTACAATAAAATGGACAATCTAAACCGCTTTAATTATT


        +50
CTTTTGTAGATATCAGTGAAACGAATGATGAAGAAGATGATGATGAAATT
GAAAACATCTATAGTCACTTTGCTTACTACTTCTTCTACTACTACTTTAA


        Sau3A+100
AGTGAAGATCATGATGATGGAGAGATAATTTTCCAAGTAGATTCAATCGA
TCACTTCTAGTACTACTACCTCTCTATTAAAAGGTTCATCTAAGTTAGCT


        +150
CGATTTTTCAATGGATTTCGAGATATTTCCTTCATTTGGCACAAGGA
GCTAAAAAGTTACCTAAAGCTCTATAAAGGAAGTAAACCGTGTTCCT
```

B, *Bam*HI     Hp, *Hpa*I    +, B/S, *Bam*HI/*Sau*3A

Bn, *Ban*II     Sa, *Sal* I     S, *Sau* 3A

E, *Eco*RI     Sm, *Sma*I

H, *Hind* III    X, *Xho* I     : A portion comprising the promoter

F i g . 6

F i g . 7  Construction of an expression plasmid  **021611**

B,BamHI;Sa.SoⅡl; E,EcoRl ; H, HindⅢ ; X ,Xhol ;

Fig. 8    Construction of lacZ expression plasmid

B , Bam HI ; S, Sau 3A ; X, Xho I ; Sa Sal I

Sm, Sma I ;

Fig. 9-1

0216117

```
          10          20          30          40          50
TCCCAACGAAATTGATGGGTTTAATAAATGGACGCCCATTTTTTACGCTG
AGGGTTGCTTTAACTACCCAAATTATTTACCTGCGGGTAAAAAATGCGAC

          60          70          80          90          100
TTCGTTCAGGTCATTCTGAAGTTATTACTGAATTATTGAAACATAATGCA
AAGCAAGTCCAGTAAGACTTCAATAATGACTTAATAACTTTGTATTACGT

          110         120         130         140         150
CGTCTGGATATTGAAGATGACAACGGCCATTCGCCACTTTTTTACGCCTT
GCAGACCTATAACTTCTACTGTTGCCGGTAAGCGGTGAAAAAATGCGGAA

          160         170         180         190         200
ATGGAGGACCACGTTGATGTTTTGAATGCACTTTTACAAGAACCATTAA
TACCCTCCTGGTGCAACTACAAACTTACGTGAAAATGTTCTTGGTAATT

          210         220         230         240         250
ATTTGCCACCGCACCCCGAATGAAATGAATTCGCAGTCTAGTACGCAACG
TAAACGGTGGCGTGGGGCTTACTTTACTTAAGCGTCAGATCATGCGTTGC

          260         270         280         290         300
CCTTAATACAATAGATTTAACCCCAAATGATGACAAATTTGATTTAGACA
GGAATTATGTTATCTAAATTGGGCTTTACTACTGTTTAAACTAAATCTGT

          310         320         330         340         350
TTCAAGATAGCATTCCGGATTTTGCTTTACCACCGCCAATCATTCCACTA
AAGTTCTATCGTAAGGCCTAAAACGAAATGCTGGCGGTTAGTAAGGTGAT

          360         370         380         390         400
AGGAAATATGGTCATAATTTTTTGGAGAAAAAATTTTCATCAAATTAA
TCCTTTATACCAGTATTAAAAAACCTCTTTTTTTAAAGTAGTTTAATTT

          410         420         430         440         450
GTTAAGGCCAGGTCTCGAGTCTATCAAGTTGATTTAGGATAACGGCATTA
CAATTCCGGTCCAGAGCTCAGATAGTTCAACTAAATCCTATTGCCGTAAT

          460         470         480         490         500
TTATGTCATCATCACCAGGCAGAATTACTTTATCCTCTAACTTACCTGAA
AATACAGTAGTAGTGGTCCGTCTTAATGAAATAGGAGATTGAATGGACTT

          510         520         530         540         550
ATAATACCGCGAAATGTTATTTTACCTGTTAGATTTGGCGAAATTAATAA
TATTATGGCGCTTTACAATAAAATGGACAATCTAAACCGCTTTAATTATT
```

Fig. 9-2

0216117

```
        560       570       580       590       600
CTTTTGTAGATATCAGTGAAACGAATGATGAAGAAGATGATGATGAAATT
GAAAACATCTATAGTCACTTTGCTTACTACTTCTTCTACTACTACTTTAA

        610       620       630       640       650
AGTGAAGATCATGATGATGGAGAGATAATTTTCCAAGTAGATTCAATCGA
TCACTTCTAGTACTACTACCTCTCTATTAAAAGGTTCATCTAAGTTAGCT

        660       670       680       690       700
CGATTTTTCAATGGATTTCGAGATATTTCCTTCATTTGGCACAAGGATAA
GCTAAAAAGTTACCTAAAGCTCTATAAAGGAAGTAAACCGTGTTCCTATT

        710       720       730       740       750
TTGCCAAAACAACGGCAATGCCATTCCTTTTCAAGAAAGTGGCAATAAAT
AACGGTTTTGTTGCCGTTACGGTAAGGAAAGTTCTTTCACCGTTATTTA

        760       770       780       790       800
AGTATTGCAACCATGAATTTACCCTTATTCCACACAAGGCTAAATAATAT
TCATAACGTTGGTACTTAAATGGGAATAAGGTGTGTTCCGATTTATTATA

        810       820       830       840       850
TGGATCTCTCACTTTGGATTACCAAATTATTTTTCCTTATCCGGGAAATC
ACCTAGAGAGTGAAACCTAATGGTTTAATAAAAGGAATAGGCCCTTTAG

        860       870       880       890       900
CACTGAAAATCATAAAATATGAGCCGTATTGGAAACTTACAGGAAGTGAT
GTGACTTTTAGTATTTTATACTCGGCATAACCTTTGAATGTCCTTCACTA

        910       920       930       940       950
TTAATGACCTCTAGTAAAGACGGTAATTTTGTTACTTCTTCATCGTTGAA
AATTACTGGAGATCATTTCTGCCATTAAAACAATGAAGAAGTAGCAACTT

        960       970       980       990       1000
TGGCAGTTTTATTAGTGTATTAGTTTGCGCTCTGAATGATGAAACCATAG
ACCGTCAAAATAATCACATAATCAAACGCGAGACTTACTACTTTGGTATC

       1010      1020      1030      1040      1050
TGGCTGCGCCAAAACCATGTGTTGAATTCAAAGGAGCGAAGATTCTGTTA
ACCGACGCGGTTTTGGTACACAACTTAAGTTTCCTCGCTTCTAAGACAAT

       1060      1070      1080      1090      1100
AATGATTTAACGAAAGAACAATTGGAAAAGTAGTGGATTATGACTTCGG
TTACTAAATTGCTTTCTTGTTAACCTTTTTCATCACCTAATACTGAAGCC
```

```
        1110      1120      1130      1140      1150
TAAAATTGATGGAAGCTTCGATGAAGTAACATTGAAACAATACTTATCTT
ATTTTAACTACCTTCGAAGCTACTTCATTGTAACTTTGTTATGAATAGAA

        1160      1170      1180      1190      1200
CGAGAGTGGTGCCTCTCAGAAGCCTACTAGAAGTTATTCCCGGGTCAGTC
GCTCTCACCACGGAGAGTCTTCGGATGATCTTCAATAAGGGCCCAGTCAG

        1210      1220      1230      1240      1250
CAGCTGGTAATTCGTGTATATTTTCCTACAGATAAGGAAATTGACACAAT
GTCGACCATTAAGCACATATAAAGGATGTCTATTCCTTTAACTGTGTTA

        1260      1270      1280      1290      1300
TCCCATCAAAATATCGCCATTTATAAATATCAATCAGTTCATTGATAAGC
AGGGTAGTTTTATAGCGGTAAATATTTATAGTTAGTCAAGTAACTATTCG

        1310      1320      1330      1340      1350
TTTTACTAATCATTTTCGAGCATGAACGTTTTTTGCGACGCAGCGGAAGT
AAAATGATTAGTAAAGCTCGTACTTGCAAAAAACGCTGCGTCGCCTTCA

        1360      1370      1380      1390      1400
TGGAGTATGCGCCAAATAGTTTTCAGTTCATGCGATTCCCAAGCTTGCTC
ACCTCATACGCGGTTTATCAAAGTCAAGTACGCTAAGGGTTCGAACGAG

        1410      1420      1430      1440      1450
GATCCTTAACTGGAAACAACCCAATTTTCCGGTTTTGTTGCAAATGAAA
CTAGGAATTGACCTTTGTTGGGTTAAAAGGCCAAAACAACGTTTACTTTT

        1460      1470      1480      1490      1500
ATCTACTCAGAGATTCAACCACAGGCAAATTTGTAGGTGATACTCCCAAT
TAGATGAGTCTCTAAGTTGGTGTCCGTTTAAACATCCACTATGAGGGTTA

        1510      1520      1530      1540      1550
TGTTTAAAGGAACTGGCAGTAAATCCTCAAAAAATGTCGTATTTAAACAC
ACAAATTTCCTTGACCGTCATTTAGGAGTTTTTTACAGCATAAATTTGTG

        1560      1570      1580      1590      1600
GGAACTAATAAACATACATACAATGGTTCAATTTGCTATGAACAATAATT
CCTTGATTATTTGTATGTATGTTACCAAGTTAAACGATACTTGTTATTAA

        1610      1620      1630      1640      1650
TGTTAGGTGTAACGCTTCCATATGAAGTACTGAAGATATGCCCGTCGTTG
ACAATCCACATTGCGAAGGTATACTTCATGACTTCTATACGGGCAGCAAC
```

Fig. 9-4

GCTAGGATCATCAAACAAAACGGACTATTGTTGATTGCATCGGTCGGGGA
CGATCCTAGTAGTTTGTTTTGCCTGATAACAACTAACGTAGCCAGCCCCT

AAATGACCAAATACCAGCTGATGGGGGTTACAGCGGGATCTACTACGCTT
TTTACTGGTTTATGGTCGACTACCCCCAATGTCGCCCTAGATGATGCGAA

GTGAGTTACTTTTTGAGAATAATATTGATATGCAAAGTTCTCTAATATGT
CACTCAATGAAAAACTCTTATTATAACTATACGTTTCAAGAGATTATACA

AGTTTTGAAATCTTATACATTATTATTGGGGAAAAACTTTTTAACCTGTT
TCAAAACTTTAGAATATGTAATAATAACCCCTTTTTGAAAAATTGGACAA

CATTAAGGGCAACATCTTCTGCCTTAGCGCCTTTATTATCTACAAGATCA
GTAATTCCCGTTGTAGAAGACGGAATCGCGGAAATAATAGATGTTCTAGT

TATTCTGCTACCATACTTTTCCACTCAATCTAATATCTAGCTGCGTCACC
ATAAGACGATGGTATGAAAGGTGAGTTAGATTATAGATCGACGCAGTGG

GTGCCCTTCAGCCAAAGCATGAAATGTGGGGCCAACCCTGCTTATCCTGC
CACGGGAAGTCGGTTTCGTACTTTACACCCCGGTTGGGACGAATAGGACG

CAATTTACAGGGCTTTTGCCCAAACCGCATAAATAGTTCAATTAATTTCA
GTTAAATGTCCCGAAAACGGGTTTGGCGTATTTATCAAGTTAATTAAAGT

AGAACCCAACCGACGCAGACTCTGTACAATGGAATTTGATTAAATTTATC
TCTTGGGTTGGCTGCGTCTGAGACATGTTACCTTAAACTAATTTAAATAG

TTTAATTCTTCGACGCACCATTTTCAATTAAAATTGGGTACGGCAAACCA
AAATTAAGAAGCTGCGTGGTAAAAGTTAATTTTAACCCATGCCGTTTGGT

TTTCTTACCAACCGCCAAGTGCAAGCTATGTAACTCCTTGATTGGTTATC
AAAGAATGGTTGGCGGTTCACGTTCGATACATTGAGGAACTAACCAATAG

Fig. 9-3

0216117

```
      2210        2220        2230        2240        2250
TTATTTTAGATCAGGCTTTAGGGGTCTGTCGTAAAGAGACTTTACCACCT
AATAAAATCTAGTCCGAAATCCCCAGACAGCATTTCTCTGAAATGGTGGA

      2260        2270        2280        2290        2300
CCAAATTCCCCACTGAACAAGCAATATGGAAAGGTGTCACATCCAAGAAT
GGTTTAAGGGGTGACTTGTTCGTTATACCTTTCCACAGTGTAGGTTCTTA
      2310        2320        2330        2340        2350
CATCTGGATAATCGTCTGGAGTTAACATTTTCCATTTTGGAAAGCAAAAA
GTAGACCTATTAGCAGACCTCAATTGTAAAAGGTAAAACCTTTCGTTTTT

      2360        2370        2380        2390        2400
ACTAGGTAATTTCATGTGCTTGGAAAGAAACTGATTAGTGTAAAGGGATT
TGATCCATTAAAGTACACGAACCTTTCTTTGACTAATCACATTTCCCTAA

      2410        2420        2430        2440        2450
CTGCCATCCTAGTCCTTCTGTAGCAACAAGGAAGGTTTGGAGTGTAGCAG
GACGGTAGGATCAGGAAGACATCGTTGTTCCTTCCAAACCTCACATCGTC

      2460        2470        2480        2490        2500
CTCTTGTACCTTGAAAAACTCATTTTCCATGCAGGCTTGATGCAAAGGAT
GAGAACATGGAACTTTTTGAGTAAAAGGTACGTCCGAACTACGTTTCCTA

      2510        2520        2530        2540        2550
AATTAGACATGGTTATACAGTTTTAAGGGCATGCGTTTATCTAGTATTTT
TTAATCTGTACCAATATGTCAAAATTCCCGTACGCAAATAGATCATAAAA

      2560        2570        2580        2590        2600
ATTATCACGCTTCAGCTGATCGTTCTTTAAGAACCCTTTTATA
TAATAGTGCGAAGTCGACTAGCAAGAAATTTCTTGGGAAAATAT
```

# DECLARATION PURSUANT TO RULE 28, PARAGRAPH 4, OF THE EUROPEAN PATENT CONVENTION

The applicant has informed the European Patent Office that, until the publication of the mention of the grant of the European patent or until the date on which the application has been refused or withdrawn or is deemed to be withdrawn, the availability of the micro-organism(s) identified below, referred to in paragraph 3 of Rule 28 of the European Patent Convention, shall be effected only by the issue of a sample to an expert.

## IDENTIFICATION OF THE MICRO-ORGANISMS

Accession numbers of the deposits:

IFO — 14456

IFO — 10207

FERM — P 8411

FERM — SP 1089